# EUROPEAN PATENT SPECIFICATION

(11) **EP 1 639 971 B1**
(45) Date of publication and mention of the grant of the patent: **24.10.2012**
(21) Application number: 03717567.6
(22) Date of filing: 11.04.2003
(51) Int. Cl.: A61K 36/899, A61K 8/97, A61K 9/00, A61K 9/20, A61K 9/70, A61L 9/16, A61Q 17/00, A61K 31/19, A23L 3/3472, A23L 1/30, A01N 65/00, A23G 3/48, A23G 4/06, A23G 9/42, A61P 31/22

(54) **SKIN PROTECTING COMPOSITIONS**
ZUSAMMENSETZUNGEN ZUM SCHUTZ DER HAUT
COMPOSITIONS POUR LA PROTECTION DE LA PEAU

(43) Date of publication of application: 29.03.2006
(73) Proprietor: Hououdou Co. Ltd., Tokyo 142-0063 (JP)
(72) Inventor: TSUCHIDA, Yuuzou, Shinagawa-Ku, Tokyo 142-0062 (JP); TSUCHIDA, Kotarou, Shinagawa-Ku, Tokyo 142-0062 (JP); TSUCHIDA, Kenjirou, Shinagawa-Ku, Tokyo 142-0062 (JP); WATANABE, Kunitomo, Gifu-Shi, Gifu 502-0816 (JP); NAKAMURA, Yoshiko, Sagamihara-Shi, Kanagawa 229-0004 (JP); IWASAWA, Atsuo, Aoba-Ku, Yokohama-Shi, Kanagawa 227-3501 (JP)
(74) Representative: Müller-Boré & Partner Patentanwälte
(86) International application number: PCT/JP2003/004635
(87) International publication number: WO 2004/091640

(56) References cited:
- EP-A- 1 304 115
- JP-A- 10 167 979
- US-A- 6 034 133
- US-A- 6 133 317
- DATABASE WPI Week 199942 Thomson Scientific, London, GB; AN 1999-496822 XP002487433 & JP 11 199501 A (NONOGAWA SHOJI KK) 27 July 1999 (1999-07-27)
- DATABASE WPI Week 200145 Thomson Scientific, London, GB; AN 2001-423285 XP002487442 & JP 2001 145660 A (TOYO INK MFG CO LTD) 29 May 2001 (2001-05-29)
- DATABASE WPI Week 198520 Thomson Scientific, London, GB; AN 1985-118636 XP002487434 & JP 60 058060 A (FIRST TRADING KK) 4 April 1985 (1985-04-04)
- SHIBATA M ET AL: "PHARMACOLOGICAL STUDIES ON BAMBOO GRASS 4. TOXICOLOGICAL AND PHARMACOLOGICAL EFFECTS OF THE EXTRACT F-III OBTAINED FROM SASA-ALBOMARGINATA" YAKUGAKU ZASSHI - JOURNAL OF THE PHARMACEUTICAL SOCIETY OF JAPAN, JP, vol. 99, no. 6, 25 June 1979 (1979-06-25), pages 663-668, XP009102793 ISSN: 0031-6903
- DATABASE WPI Week 200369 Thomson Scientific, London, GB; AN 2003-725421 XP002487445 & JP 2003 201247 A (TSUCHIDA K) 18 July 2003 (2003-07-18)
- KUBO M. ET AL.: 'Kumazasa hasen'i kakikomi nuno' no atopy-sei hifuen kanja eno oyo' NIHON YAKUGAKKAI NENKAI KOEN YOSHISHU vol. 122, no. 2, 2002, page 165, XP002986459
- KONDO I. ET AL.: 'Kumazasa chushutsueki no kiiro budokyukin narabi no katabetsu budokyukin phage no zoshoku ni oyobosu eikyo' JAPANESE JOURNAL OF BACTERIOLOGY vol. 55, no. 2, 2000, page 341, XP002986460

## Description

The present invention relates to a composition for use in preventing and/or treating a viral infection of herpes virus.

Contagious bacteria such as Pseudomonas aeruginosa, Bacillus subtilis, Bacillus anthracis, Escherichia coli, Staphylococcus aureus, and viruses such as the influenza virus float freely in the air, can intrude into the body from the mucosa and wound regions of humans and animals, and can cause severe infections in humans and animals. A variety of antibiotics are used for this kind of microbial infection. However, various kinds of antibiotic resistant bacteria such as methicillin-resistant staphylococcus aureus (MRSA), against which conventional antibiotics are not effective, have appeared as a result of the overuse of antibiotics. This results in patients, doctors and nurses in hospitals becoming infected with resistant bacteria, sometimes even leading to death.

Meanwhile, a mail containing anthrax was sent recently to politicians and mass media-related personalities in the U.S., and many of the unprotected people who opened the mail were infected with anthrax leading to mortalities, and causing widespread fear in society. Then, the desirability of having a suitable protective means against this kind of biological weapon not only for those preparing for public work, but also for general citizens quickly became apparent.

Consequently, an object of the present invention is to provide a skin protection composition that can effectively suppress intrusion into the body and growth therein of herpes virus from skin, wounds and from mucosa such as those of the eyes, nose, throat, ears, anus and vagina; i.e.

to provide a preventive and/or therapeutic composition for virus infections of herpes virus.

The present invention provides the following skin protection compositions, preventive and/or therapeutic compositions for virus infection.
1. A composition comprising for use in preventing and/or treating a viral infection of herpes virus Sasa extract as an active component and
2. The composition according to the above 1 that is in the form of an oral composition.
3. The composition according to 2 above that is in the form of a confection such as a gummi, jelly, troche, candy, chewing gum, jam, sherbet, cream, drop, sponge cake, cookie, chocolate, or rice cracker, breads, noodles, beverages, tablets, pills, mouthwash, gargle, toothpaste, or skin adhesive film.
4. The composition according to any one of the above 1 that is in the form of a protective cloth.
5. The composition according to 4 above that is in the form of gauze, mask, eye patch, menstrual band, menstrual napkin, medical dressing, toilet paper, gauze for treating hemorrhoids, toilet seat sheet, shoe insert, ear plug, glove, hat, white gown, sheet, futon cover, pillow case, curtain, wall paper, carpet, and surgical suture thread.
6. The composition according to 4 above that is in the form of gauze.
7. A mask comprising the gauze according to 6 above.

The present invention will be explained in detail below.

### Sasa extract

The Sasa (Sasa albo-marginata) used in the present invention, as a raw material for the Sasa extract is not restricted to any specific one and any plant belonging to the genus Sasa may be used herein. Specific examples thereof include those specified below: Kumai Sasa; Sasa albo-marginata Makino et Shibata (Kuma Sasa); ground bamboo; Okuyama Sasa; Ezo-Miyama Sasa; Sasa Paniculata Makino et Shibata; Yahiko Sasa; Oba Sasa; Miyama Sasa; Sendai Sasa; Yukawa Sasa; Aboi Sasa; and Onuka Sasa. Among these, specific examples of commercially available ones include Kumai Sasa and Kuma Sasa (Chugoku Sasa and Hida Sasa). For instance, preferably used herein are extracts derived from, for instance, Kumai Sasa and/or Kuma Sasa collected in, for instance, TESHIO Mountains in Hokkaido, Japan during the term extending from July to October. The extract used in the present invention is preferably obtained by normal pressure or pressurized extraction from fresh or dried leaves, preferably dried leaves, in 100 to 180°C water.

The extraction method is not particularly limited, but may be, for example, the method described in Japanese Patent No. 3212278 (Japanese Unexamined Patent Application Publication No. H11-196818). Concretely, an extract is extracted by processing 5 to 30 minutes at 100 to 180°C using a pressurized hot water extractor, and separating the extract in question from the moist solid parts (water content percentage 40 to 70%) using a water content separator. After using a saturated water steam heat processor to process the moist solid part at 100 to 200°C for 5 to 60 minutes, the extract is again extracted by processing 5 to 30 minutes at 100 to 180°C using a pressurized hot water extractor, and the first and second extracts are combined and used. An extract obtained by extracting from Sasa dried leaf in 60 to 100°C water for about 30 minutes to 12 hours may also be used.

"AHSS" manufactured by Chloroland Moshiri Co., Ltd. is an example of a commercial product comprising 50% solid weight Sasa extract.

The Sasa extract obtained in this way comprises a sulfur component, and converted to sulfur, the content is approximately 4 to 10 mg per gram of solid weight Sasa extract, normally, 6 to 9 mg. The primary component of this sulfur constituent appears to be amino acid containing sulfur.

The composition of the present invention per 100 g solid extract preferably contains 4 to 500 mg of sulfur component derived from Sasa extract converted to sulfur; more preferably 8 to 250 g; and most preferably, 16 to 150 mg.

Moreover, Sasa extract contains tannin, and that content is about 5 to 15% by mass in relation to the solid content of the Sasa extract.

The composition of the present invention preferably contains 0.05 to 7.5% Sasa tannin by mass in dry component concentration, and more preferably 0.1 to 6% by mass.

The composition of the present invention has Sasa extract as the active component, and further suitable amount of organic acid. Malic acid, citric acid, lactic acid, oxalic acid, malonic acid, succinic acid, fumaric acid, acetic acid, benzoic acid, phenylacetic acid, salicylic acid, and phenols may be cited as this kind of organic acid.

The amount of organic acid used is preferably 0.01 to 5% by mass in the composition of the present invention; more preferably 0.02 to 3% by mass, and most preferably 0.05 to 1.5% by mass.

### Skin protection composition, viral infection preventive and/or therapeutic composition

The skin protection composition, viral infection preventive and/or therapeutic composition of the present invention is a substance that effectively suppresses intrusion into the body of contagious bacteria and viruses from skin, from wounds and from mucous membranes such as those of the eyes, nose, throat, ears, anus and vagina, and effectively prevents and/or treats herpes virus.

Skin protective cloth and oral compositions of the present invention may be cited as forms of the skin protection composition comprising Sasa extract as the active component, as well as of compositions to prevent and/or treat viral infections.

Skin protective cloth comprises an air permeable support impregnated or sprayed with Sasa extract, the air permeable support including, for example, natural fibers such as silk, cotton, hemp or wool, synthetic fibers such as polyurethane, vinylon, polypropylene, nylon, polyester, or acrylic, semi-synthetic fibers, or fibers themselves, or threads, woven cloth, knitted cloth, non-woven cloth, or paper which is a mixture of two or more kinds of these. As a matter of convenience, in addition to cloth, "protective cloth" in the present specification shall include the fibers themselves, thread, paper or the like.

In addition to protective cloth that directly contacts mucosa and skin and is classified as a hygienic product such as gauze, masks, eye patches, menstrual bands, menstrual napkins, medical dressings, toilet paper, , toilet seat sheets, shoe inserts, ear plugs, and plasters, examples of concrete forms include clothing such as white gowns, clothing items such as gloves, hats, socks, and Japanese socks, bedding such as sheets, futon covers, pillow cases, and bed articles, room interior accessories and materials such as curtains, wall paper and carpets, medical materials such as surgical suture thread, articles that directly or indirectly touch the skin, and cooking utensils such as chopping blocks.

Oral compositions comprising Sasa extract of the present invention include, for example, confections such as gummis, jellies, troches, candies, chewing gums, jams, sherbets, creams, drops, sponge cakes, cookies, chocolates, or rice crackers, breads, noodles, beverages, tablets, pills, mouthwash, gargle, toothpaste, or skin adhesive film,

To include Sasa extract in the skin protective cloth, the protective cloth is immersed in an aqueous solution of 2 to 20% Sasa extract by mass, or an aqueous solution of 2 to 20% Sasa extract by mass is sprayed on the protective cloth and dried. For a suitable amount of impregnated or sprayed Sasa extract, it is preferable to contain 2 to 20% Sasa extract by mass in the solid content, more preferably 6 to 15% by mass, and most preferably 8 to 12% by mass. If less than 2% by mass, the targeted effect will be insufficiently manifested; and if exceeding 20% by mass, the skin or mucosa may experience a burning sensation, and with little further improvement in effect there will be no economic advantage.

Per 100 g of protection composition, the skin protection composition of the present invention preferably contains 8 to 200 mg of sulfur component derived from Sasa extract converted to sulfur, more preferably 24 to 150 mg, and most preferably 32 to 120 mg.

To include Sasa extract in oral compositions such as, for example, in a confection such as a gummi, jelly, troche, candy, chewing gum, jam, sherbet, cream, drop, sponge cake, cookie, chocolate, or rice cracker, breads, noodles, beverages, tablets, pills (for example, "Sasatan"), mouthwash, gargle, toothpaste, of skin adhesive film, at every stage of manufacturing the oral composition, it is preferable to add 2 to 20% Sasa extract solid content by mass to the raw materials of the oral composition, more preferably 6 to 15% by mass, and most preferably 8 to 12% by mass. If less than 2% by mass, the targeted effect will be insufficiently manifested; and if exceeding 20% by mass, the skin or mucosa may experience a burning sensation, and with little further improvement in effect there will be no economic advantage.

When including, or after including the Sasa extract in the protective cloth or oral composition, it is preferable to heat process at a temperature of 70 to 90 °C, more preferably, of 75 to 85°C, for 30 minutes to 3 hours, more preferably, for 1 to 2 hours. Heat processing dramatically improves the skin protection effect. Suitable and customary components may be compounded in the oral composition of the present invention depending on the dosage form. For example, excipients such as fructose, lactose, sucrose, starch syrup, dextrin, and cyclodextrin, binders such as gum Arabic, sodium carboxymethyl cellulose, crystalline cellulose, and gum base, disintegrating agents such as starch, lubricants such as magnesium stearate, and sucrose fatty acid esters, and fresheners such as fragrance, chlorophyll, spearmint, mint, and menthol may be cited.

When using the skin protection composition of the present invention in the form of a protective cloth, Sasa extract is incorporated in the protective cloth (gauze, or the like) at the location that contacts the mucosa or skin region, and this protective cloth may be replaced about 1 to 3 times a day. When using in the form of surgical suture thread, intrusion of bacteria from the sutured wound area is suppressed, infection of the wound area is suppressed, and the healing of the surgical region is promoted.

If the skin protection composition of the present invention is used in the form of a protective cloth in order to prevent hospital infection at clinics or the like, the infection prevention effect of the skin protection composition of the present invention will continue for a long period of time. When that effect is reduced by laundering, the protective cloth may be suitably replaced or re-treated to impregnate Sasa extract again.

If the skin protection composition of the present invention is used in the form of an oral composition, the amount ingested is not particularly limited, but about 2 to 15 mg as Sasa extract solid content may be ingested, for example 1 to 5 times daily, normally 1 to 3 times. The amount and frequency of ingestion may be suitably increased or decreased to match objectives.

The skin protection composition of the present invention contains 2 to 20% Sasa extract solid content by mass, and indicates a marked antibacterial effect on resistant bacteria for which conventional antibiotics are not effective.

In addition, when using the skin protection composition of the present invention in the form of an oral composition, contagious bacterial viral infections can be prevented and the growth thereof can be suppressed in an extremely simple manner by taking the composition orally, if suitable and necessary. In addition, gradual release forms of the composition such as chewing gum and candy have the advantage of manifesting that effect over a prolonged period of time.

Skin protection compositions of the present invention can be used in the form of ointments, creams, hair gels, gels, lotions, oils, soaps, and shampoos. Viral infections can be prevented and the growth thereof can be suppressed in an extremely simple manner by coating the skin with ointment, cream, hair gel, gel, lotion or oil (for example, jojoba oil, grape seed oil, and oryza oil) containing 2 to 20% Sasa extract solid content by mass.

### Antiseptic

The present invention also provides an antiseptic containing Sasa extract as the active component. The form when used as an antiseptic is not particularly limited. Food, beverages, condiments, cosmetics (including lotions and oils), sprays for treatment of wounds, and sprays for treatment of throat inflammations or the like may be cited as antiseptic compositions. It is preferable that 2 to 20% Sasa extract solid content by mass is contained in these target substances, more preferably 6 to 15% by mass, and most preferably 8 to 12% by mass. If less than 2% by mass, the targeted antiseptic effect will be insufficiently manifested; and if exceeding 20% by mass, there will be little further improvement in effect, and no economic advantage. In addition, if adding to foods, an undesirable change in the flavor of the food may occur.

Reference examples, embodiments and test examples are indicated below to explain the present invention in further detail, but naturally, the present invention is not limited to these. "%" is "% by mass" in this specification unless otherwise noted.

### Reference example 1: Preparation of Sasa extract

Dried leaves of Kumazasa collected in Teshio Mountains in Hokkaido Japan in September were introduced into a pressurized hot water extraction tank, treated at 125°C for 10 minutes in the tank, the hot water was cooled down to about 80°C by the action of a cooling water and then the resulting extract was separated from the moisture-containing solid content using a screw-press in such a manner that the moisture content of the latter was controlled to a level of about 50% by mass. Then the solid contents having a moisture content of about 50% by mass were introduced into an autoclave and heat-treated under pressure at 180°C for 10 minutes using saturated steam. The moisture-containing solid contents thus treated were again introduced into a pressurized hot water-extraction tank and treated at 110°C for 5 minutes to thus obtain an extract. The first and second extracts were combined, filtered through a diatomaceous earth layer, the resulting filtrate was concentrated under reduced pressure until the solid content thereof was increased to 50% by mass and the concentrate thus prepared was subjected to flow sterilization treatment at a temperature ranging from 110 to 130°C to yield a Sasa extract.

The sulfur content of the Sasa extract thus prepared was 3850 *µ* g/ml (7.7 mg/gram of solid content).

### Reference Example 2: Sasa extract containing organic acids

Sasa extract containing organic acid was produced by adding 0.5 g, 1 g, 1.5 g or 2 g of malic acid per 100 g of the Sasa extract (solid content 50% by mass) produced in Reference Example 1.

### Reference Embodiment 1 Production of chewing gum

Two to fifteen grams of the Sasa extract (solid content 50% by mass) produced in Reference Example 1 was added 100 g gum base heated to 60°C for 30 minutes in a constant temperature bath, kneaded for 2 minutes, and reheated to 60°C for 10 minutes. The kneaded mixture thus obtained was left to cool, and then was rolled to produce stick chewing gum with a thickness of approximately 1 mm (as well as chewing gum in a variety of shapes including balls).

### Reference Embodiment 2 Production of pills

A compound of the following composition (units in % by mass) was uniformly mixed in a kneader, and then was formed into pills in a granulator.

| | |
|---|---|
| Sasa extract of Reference Example 1 (solid content 50% by mass) | 20 |
| Gambir | 10 |
| Licorice | 15 |
| Cassia | 15 |
| Fennel | 5 |
| Mint | 5 |
| Thyme | 5 |
| Ginger | 5 |
| Clove | 5 |
| Hydrangea (Amacha) | 5 |
| Cornstarch | 8 |
| Magnesium oxide | 2 |
| Total | 100 |

### Reference Embodiment 3 Protective gauze

A solution of Sasa extract (solid content 50% by mass) produced in Reference Example 1 diluted 10 times was sprayed on silk gauze, and was dried for 1 hour at 80°C to produce protective gauze containing 8.8% Sasa extract solid content by mass.

### Embodiment 1 Protective gauze

Silk gauze was immersed in a 6-fold aqueous dilution solution of Sasa extract (solid content 50% by mass) produced in Reference Example 2 for one hour at 80°C, and was dried for one hour at 80°C to produce protective gauze containing 8% Sasa extract solid content by mass.

### Embodiment 2 Protective masks

Protective masks were produced by inserting the protective gauzes of Reference Embodiment 3 and Embodiment 1 on the inside of masks comprising multiple layers of gauze.

### Embodiment 3 Air cleaner

A filter used in an ordinary air cleaner was immersed in a 6-fold aqueous dilution solution of Sasa extract (solid content 50% by mass) produced in Reference Example 2 for one hour at 80°C, and was dried for one hour at 80°C to produce a filter containing 8% Sasa extract solid content by mass. This was installed in an air cleaner.

### Reference Test Example 1 Antibiotic tests

The protective gauze produced in Reference Embodiment 3 (containing Sasa extract solid content 8.8% by mass) and silk gauze not containing Sasa extract (control gauze) were used.

One milliliter of the following bacterial solutions (bacteria count approximately 5.0x10⁴ bacteria/mL or approximately 5.0x10⁵ bacteria /mL) were dripped on the respective gauzes (5x7 cm), and were left to stand and cultured for 12 hours at 37°C in an incubator. After culturing was complete, the gauze was thoroughly rinsed with 10 mL of liquid culture medium to produce the test solution. A spiral system (NASA system, USA) was used to coat this on a brain heart agar plate culture medium. The plate was left to stand and cultured for 18 hours at 37°C in an incubator. The colonies on the brain heart agar plate were counted, and the results are indicted in Table 1 and Table 2.

**Table 1**

| Bacteria | Number of bacteria inoculated | Bacteria count after storage | |
|---|---|---|---|
| | | Gauze of the present invention | Control gauze |
| Staphylococcus aureus | 5.0x10⁴ | 2.0x10(<10²) | >10⁶ |
| MRSA | 5.1x10⁴ | 1.0x10 (<10²) | >10⁶ |
| Pseudomonas aeruginosa | 5.6x10⁴ | 5.0x10(<10²) | > 10⁶ |
| Escherichia coli | 5.2x10⁴ | 8.0x10(<10²) | >10⁶ |
| Bacillus subtilis | 5.4x10⁴ | 9.0x10(<10²) | >10⁶ |

**Table 2**

| Bacteria | Number of bacteria inoculated | Bacteria count after storage | |
|---|---|---|---|
| | | Gauze of the present invention | Control gauze |
| Staphylococcus aureus | 5.4x10⁵ | 5.0x10(10²) | >10⁷ |
| MRSA | 5.5x10⁵ | 6.0x10(<10²) | >10⁷ |
| Pseudomonas aeruginosa | 5.6x10⁵ | 9.0x10(<10²) | >10⁷ |
| Escherichia coli | 5.2x10⁵ | 9.8x10² | >10⁷ |
| Bacillus subtilis | 5.4x10⁶ | 8.2x10² | >10⁷ |

The numbers in parenthesis in Tables 1 and 2 indicate the bacteria count by general display.

In contrast to the decrease of bacteria count of the gauze containing Sasa extract of the present invention to approximately 1 in 1000 to 1 in 10000 after 12 hours of culturing, the bacteria count of the gauze not containing Sasa extract increased 100 times or more. This indicates that the gauze containing Sasa extract of the present invention has superior antibacterial characteristics, and specifically has notable antibacterial characteristics in relation to resistant bacteria MRSA on which antibiotics have no effect.

### Reference Test Example 2 Growth suppression tests on anthrax bacteria

### 1. Test method

Strain used: Spore solution of anthrax 34-F2 (toxogenic, encapsulated asporogenous weakened strain). This was adjusted to 34,000 bacteria per 0.1 mL.
Test solution: Solution of Sasa extract solid content 50% by mass
Investigation method: The aforementioned test solution was diluted with sterile distilled water, and adjusted to solutions of 25%, 12.5%, 6.25%, 3.2%, 1.6%, 0.8%, and 0.4%, and investigations were conducted in the following two ways. Sterile distilled water was used as the control.
   1) After mixing 0.1 mL of anthrax 34-F2 spore solution in 1 mL each of the 10 types of 50 to 0% test solutions prepared above and leaving to stand for 24 hours at 37°C, 0.1 mL of this was smeared on brain heart infusion (BHI) agar medium, and after culturing for 24 hours at 37°C, colony growth was observed. The increase or decrease of bacteria count in sterile distilled water was also observed at this time.
   2) A two-fold concentration of BHI agar was added to 10 mL each of the 10 types of 50 to 0% test solutions produced above to prepare agar plates. 0.1 mL of anthrax 34-F2 spore solution was smeared on the respective media, and after culturing for 24 hours at 37°C, the colony growth was observed.

### 2. Results

The results are indicated in Table 3 below.

**Table 3**

| Sasa extract solid component concentration (%) | | | | | | | | | |
|---|---|---|---|---|---|---|---|---|---|
| 25 | 12.5 | 6.25 | 3.2 | 1.6 | 0.8 | 0.4 | 0.2 | 0.1 | 0 |
| - | - | - | - | - | - | +/- | + | + | + |

| | | | | | | | | | |
|---|---|---|---|---|---|---|---|---|---|
| +: Colonies grow with no difference from the control -: No colonies observed +/-: Colony formation is observed, but there are fewer than with the control. | | | | | | | | | |

### 3. Conclusions

It was not confirmed that the tested Sasa extract has a power to sterilize anthrax spores, but Sasa extract did strongly suppress the growth of anthrax bacteria. As a result, the skin protective composition of the present invention containing Sasa extract can suppress the growth of anthrax bacteria, and consequently can prevent infection by anthrax bacteria.

### Reference Example 3 Test of antiviral effect on the influenza virus

### 1. Test method

Herpes simplex virus (HSV) HF strain, herpes simplex virus (HSV) UW strain, influenza virus (Inf.) A/PR/8 strain, and influenza virus (Inf.) A/FM/1/47 strain were used. Vero cells (derived from African green monkey kidney) were used as the culture cells for HSV, and MDCK cells (derived from dog liver) were used for the Inf.

After mixing 10 µL of viral solution with 1 mL of Sasa extract produced in Reference Example 1 diluted with sterile distilled water, the established cell line cultured at room temperature (23±3°C) in a 24-well plate was inoculated with 10 µL of viral solution. This plate was cultured in a CO₂ incubator at 37°C, and the antiviral effect was judged by the extent of cytopathic effect (CPE) based on the following criteria. Distilled water (DW) was used as the control.

NT: not tested; 4+ CPE on entire surface of well; 3+ CPE on 75% or more of the well; 2+: CPE on 50 to 75% of the well; +: CPE on 25 to 50% of the well; ±: CPE on 25% or less of the well; -: CPE not observed.

For electron microscope samples, the virus was allowed to infect the culture cells for 20 hours; the cells were treated with dilute Sasa extract, and then were fixed and observed following standard methods.

### 2. Results

The antiviral effects on herpes simplex virus (HSV) HF strain, herpes simplex virus (HSV) UW strain, influenza virus (Inf.) A/PR/8 strain, and influenza virus (Inf.) A/FM/1/47 strain are indicated in Tables 4 to 7.

**Table 4 HSV HF strain**

| | Sasa extract solid component concentration (%) | | | | | | |
|---|---|---|---|---|---|---|---|
| | 10 | 8 | 5 | 3 | 2 | 1 | Distilled water |
| 1 minute | 3+ | 4+ | 4+ | 4+ | 4+ | 4+ | 4+ |
| 5 minutes | - | ± | 4+ | 4+ | 4+ | 4+ | 4+ |
| 10 minutes | - | - | - | 2+ | 4+ | 4+ | 4+ |
| 15 minutes | | - | - | - | ± | 2+ | 4+ |
| 30 minutes | - | - | - | - | - | - | 4+ |
| 60 minutes | - | - | - | - | - | - | 4+ |

The virus was deactivated in 5 minutes processing at a concentration of 10%, and was deactivated in 1 hour of processing even at 1% concentration.

**Table 5 HSV UW strain**

| | Sasa extract solid component concentration (%) | | | | | | |
|---|---|---|---|---|---|---|---|
| | 10 | 8 | 5 | 3 | 2 | 1 | Distilled water |
| 1 minute | - | ± | 2+ | 2+ | 3+ | 4+ | 4+ |
| 5 minutes | - | - | 1+ | ± | 2+ | 3+ | 4+ |
| 10 minutes | - | - | - | - | ± | ± | 4+ |
| 15 minutes | - | - | - | - | - | - | 4+ |
| 30 minutes | - | - | - | - | - | - | 4+ |
| 60 minutes | - | - | - | - | - | - | 4+ |

The virus was deactivated in 1 minute processing at a concentration of 10%, and was deactivated in 15 minutes of processing even at 1% concentration.

**Table 6 Inf. A/PR/8 strain**

| | Sasa extract solid component concentration (%) | | | | | |
|---|---|---|---|---|---|---|
| | 5 | 2 | 1 | 0.5 | 0.1 | Distilled water |
| 1 minute | 4+ | 4+ | 4+ | 4+ | 4+ | 4+ |
| 5 minutes | 4+ | 4+ | 4+ | 4+ | 4+ | 4+ |
| 10 minutes | 2+ | 2+ | 2+ | 2+ | 4+ | 4+ |
| 15 minutes | 2+ | 4+ | 2+ | 2+ | 4+ | 4+ |
| 30 minutes | - | - | - | - | 2+ | 4+ |
| 60 minutes | - | - | - | - | 2+ | 4+ |

The virus was deactivated in 30 minutes processing at a concentration of 0.5% or greater.

**Table .7 Inf. A/FM/47 strain**

| | Sasa extract solid component concentration (%) | | | | | |
|---|---|---|---|---|---|---|
| | 5 | 2 | 1 | 0.5 | 0.1 | Distilled water |
| 1 minute | 4+ | 4+ | 4+ | 4+ | 4+ | 4+ |
| 5 minutes | 4+ | 4+ | 4+ | 4+ | 4+ | 4+ |
| 10 minutes | 4+ | 4+ | 2+ | + | 2+ | 4+ |
| 15 minutes | 4+ | 4+ | - | + | 2+ | 4+ |
| 30 minutes | 4+ | 4+ | - | - | ± | 4+ |
| 60 minutes | 2+ | 2+ | - | - | - | 4+ |

The virus was deactivated in 30 minutes processing at a concentration of 0.5% or greater.

### 3. Conclusions

These results demonstrate that the anti-viral effect of Sasa extract on the influenza virus differs from that of existing preparations, but the mechanism of action is unclear.

## Claims

1. A composition for use in preventing and/or treating a viral infection of herpes virus, comprising Sasa extract as an active component and further an organic acid.

2. The composition for use according to claim 1 that is in the form of an oral composition.

3. The composition for use according to claim 2 that is in the form of a confection such as a gummi, jelly, troche, candy, chewing gum, jam, sherbet, cream, drop, sponge cake, cookie, chocolate, or rice cracker, breads, noodles, beverages, tablets, pills, mouthwash, gargle, toothpaste, or skin adhesive film .

4. The composition for use according to claim 1 that is in the form of a protective cloth.

5. The composition for use according to claim 4 that is in the form of gauze, mask, eye patch, menstrual band, menstrual napkin, medical dressing, toilet paper, toilet seat sheet, shoe insert, ear plug, glove, hat, white gown, sheet, futon cover, pillow case, curtain, wall paper, carpet, and surgical suture thread.

6. The composition for use according to claim 4 that is in the form of gauze.

7. A mask for use according to claim 1 gauze as a protective, which incorporates a composition comprising sasa extract as an active component and further an organic acid.

## Patentansprüche

1. Zusammensetzung zur Verwendung in der Prävention und/oder Behandlung einer viralen Infektion mit Herpesvirus, umfassend Sasa-Extrakt als einen aktiven Bestandteil und weiter eine organische Säure.

2. Zusammensetzung zur Verwendung nach Anspruch 1, die in Form einer oralen Zusammensetzung vorliegt.

3. Zusammensetzung zur Verwendung nach Anspruch 2, die in Form einer Zubereitung wie ein Gummi, Gelee, Pastille, Bonbon, Kaugummi, Marmelade, Sorbet, Creme, Drops, Biskuitkuchen, Keks, Schokolade oder Reiskracker, Brot, Nudeln, Getränken, Tabletten, Pillen, Mundwasser, Gurgelmittel, Zahnpasta oder Klebfolie für die Haut vorliegt.

4. Zusammensetzung zur Verwendung nach Anspruch 1, die in Form eines schützenden Stoffs vorliegt.

5. Zusammensetzung zur Verwendung nach Anspruch 4, die in Form von Gaze, Maske, Augenklappe, Monatsbinde, Monatseinlage, medizinischem Verband, Toilettenpapier, Toilettensitzabdeckung, Schuheinlage, Ohrenstöpsel, Handschuhe, Hut, weißem Kittel, Laken, Futonabdeckung, Kissenbezug, Vorhang, Tapete, Teppich und chirurgischem Nähfaden vorliegt.

6. Zusammensetzung zur Verwendung nach Anspruch 4, die in Form von Gaze vorliegt.

7. Maske zur Verwendung nach Anspruch 1, umfassend Gaze als einen schützenden Stoff, das eine Zusammensetzung, umfassend Sasa-Extrakt als einen aktiven Bestandteil und weiter eine organische Säure, enthält.

## Revendications

1. Composition pour utilisation dans la prévention et/ou le traitement d'une infection virale par le virus de l'herpès, comprenant un extrait de Sasa en tant que substance active et en outre un acide organique.

2. Composition pour utilisation selon la revendication 1 qui est sous la forme d'une composition orale.

3. Composition pour utilisation selon la revendication 2 qui est sous la forme d'une confiserie telle qu'une gomme, une gelée, une pastille, un bonbon, une gomme à mâcher, une confiture, un sorbet, une crème, une goutte, une éponge, un gâteau, un biscuit, un chocolat, ou des craquelins au riz, des pains, des nouilles, des boissons, des tablettes, des pilules, un bain de bouche, un gargarisme, un dentifrice ou un film adhésif cutané.

4. Composition pour utilisation selon la revendication 1 qui est sous la forme d'une toile de protection.

5. Composition pour utilisation selon la revendication 4 qui est sous la forme d'une gaze, un masque, une pièce oculaire, une bande menstruelle, une serviette hygiénique, un pansement médical, un papier toilette, une feuille de protection pour siège de toilettes, un insert pour chaussure, un bouchon d'oreille, un gant, un chapeau, une blouse blanche, un drap, une housse de futon, une housse de coussin, un rideau, un papier peint, un tapis, et un fil de suture chirurgicale.

6. Composition pour utilisation selon la revendication 4 qui est sous la forme d'une gaze.

7. Masque pour utilisation selon la revendication 1 comprenant une gaze en tant que toile de protection, qui incorpore une composition comprenant un extrait de Sasa en tant que substance active et en outre un acide organique.
